# EUROPEAN PATENT SPECIFICATION

(11) **EP 1 934 212 B1**
(45) Date of publication and mention of the grant of the patent: **20.02.2013**
(21) Application number: 06793916.5
(22) Date of filing: 29.09.2006
(51) Int. Cl.: C07D 451/14, A61K 31/439, A61P 25/24

(54) **9-AZABICYCLO [3 . 3 . 1]NONANE DERIVATIVES AS MONOAMINE REUPTAKE INHIBITORS**
9-AZABICYCLO[3 . 3 . 1]NONANDERIVATE ALS MONOAMINWIEDERAUFNAHMEHEMMER
DÉRIVÉS DE 9-AZABICYCLO[3 . 3 . 1]NONANE EN TANT QU'INHIBITEURS DU RECAPTAGE DES MONOAMINES

(30) Priority: 30.09.2005 EP 05109123
(43) Date of publication of application: 25.06.2008
(73) Proprietor: MSD Oss B.V., 5349 AB Oss (NL)
(72) Inventor: BINGHAM, Matilda, Jane, Newhouse ML1 5SH (GB); HUGGETT, Margaret, Jean, Central Scotland ML1 5SH (GB); HUGGETT, Mark, Central Scotland ML1 5SH (GB); KIYOI, Yasuko, Central Scotland ML1 5SH (GB); NAPIER, Susan, Elizabeth, Lanarkshire ML1 5SH (GB); NIMZ, Olaf, Central Scotland ML1 5SH (GB)
(74) Representative: Jaap, David Robert
(86) International application number: PCT/EP2006/066896
(87) International publication number: WO 2007/039563

(56) References cited:
- EP-A- 1 403 255
- WO-A-2004/113334
- US-A- 5 958 945
- PATENT ABSTRACTS OF JAPAN vol. 016, no. 544 (C-1004), 13 November 1992 (1992-11-13) -& JP 04 208267 A (MITSUI PETROCHEM IND LTD; others: 01), 29 July 1992 (1992-07-29) & DATABASE WPI Section Ch, Week 199237 Derwent Publications Ltd., London, GB; Class B02, AN 1992-303575 XP002361888
- KRAISS G ET AL: "CHEMISTRY OF TROPAN-3-YL ETHERS. I. SYNTHESIS OF TROPAN-3-YL ETHERS" JOURNAL OF THE CHEMICAL SOCIETY, SECTION B: PHYSICAL ORGANIC CHEMISTRY, CHEMICAL SOCIETY. LETCHWORTH, GB, no. 11, 1971, pages 2145-2149, XP009055570 cited in the application

## Description

The present invention relates to a 9-azabicyclo[3.3.1]nonane derivative, to a pharmaceutical compositions comprising said compound and to its use in therapy, in particular to its use for the treatment or prevention of a disease or disorder for which the reuptake inhibition of one or more monoamine neurotransmitter contributes to the therapeutic effect.

Monoamine reuptake inhibitors have found widespread use in therapy, in particular, in the treatment of depression, a common, serious and life-threatning disorder with persistant and debilitating side-effects. The older tricyclic monoamine reuptake inhibitors, including imipramine and amitriptyline are effective antidepressants but these compounds additionally have deleterious cardiovascular and anticholinergic side-effects which can lead to serious toxicity in overdose and to poor patient compliance. The newer drugs, such as the selective serotonin reuptake inhibitors (SSRIs), whilst being an improvement over older antidepressants have their own particular pattern of side-effects which include sleep disturbances, gastrointestinal symptoms and sexual problems. Monoamine reuptake inhibitors are also indicated to be useful in the treatment of other disorders such as pain, panic disorders and obsessive compulsive disorder.

In view of the shortcomings of the currently available monoamine reuptake inhibitors, the search for new compounds which are safe and effective continues. In particular, recently, there has been renewed interest in a group of drugs which in addition to inhibiting the reuptake of serotonin, also inhibit the reuptake of noradrenaline and dopamine.

WO 04/113334 discloses 8-azabicyclo[3.2.1]octane derivatives indicated to be monoamine neurotransmitter reuptake inhibitors and as such useful in the treatment of diseases or disorders responsive to inhibition of monoamine neurotransmitter reuptake in the central nervous system. WO 04/113334 however does not disclose ring systems apart from 8-azabicyclo[3.2.1]octane.

WO 03/062235 discloses thio-bridged aryl compounds, including 3-arylthio-9-azabicyclo[3.3.1]nonane derivatives, indicated to be modulators of acetylcholine receptors and as such, useful for the treatment of dysfunctions of the central and autonomic nervous system. WO 03/062235 however does not disclose 3-aryloxo-9-azabicyclo[3.3.1]nonane derivatives or 3-arylamino-9-azabicyclo[3.3.1]nonane derivatives.

WO 02/100833 discloses heterocyclic compounds indicated to be useful as therapeutic agents for diseases for which Rho kinase is responsible. There is no suggestion, however, that the compounds disclosed in WO 02/100833 would be useful for the manufacture of a medicament for the treatment or prevention of a disease or disorder for which the reuptake inhibition of one or more monoamine neurotransmitter contributes to the therapeutic effect. US 5,958,945 relates to 3-aminoazabicyclooctane or nonane derivatives useful as neuroleptic agents. US 5,958,945, however, does not deal with 3-aryloxy derivatives.

WO 2005/123728 discloses generically 9-azabicyclo[3.3.1]nonane derivatives having a 3-aryloxy substituent. WO 2005/123728, however, does not disclose any specific 9-azabicyclo[3.3.1] nonane derivatives having a 3-aryloxy substituent, wherein the aryl group is a heteroaryl ring selected from benzothienyl and benzoisothiazolyl, said heteroaryl ring being optionally substituted with chloro, fluoro or methyl.

The synthesis of exo-9-methyl-3-phenoxy-9-azabicyclo[3.3.1]nonane is described in J. Chem. Soc., Phys. Org., 1971, 11, 2145. No suggestion however is made in J. Chem. Soc., Phys. Org., 1971, 11, 2145 about any possible medicinal properties of said compound.

In a first aspect the present invention provides a 9-azabicyclo[3.3.1]nonane derivative of formula I 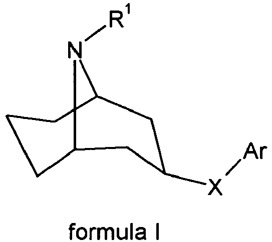 wherein
R¹ is H;
X is O and
Ar is a heteroaryl ring selected from benzothienyl and benzoisothiazolyl, said 9-azabicyclo[3.3.1]nonane derivative being selected from:
*exo*-3-(benzo[d]isothiazol-7-yloxy)-9-azabicyclo[3.3.1]nonane;
*exo*-3-(benzo[d]isothiazol-4-yloxy)-9-azabicyclo[3.3.1]nonane;
*exo-*3-(benzo[b]thiophen-7-yloxy)-9-azabicyclo[3.3.1]nonane;
*exo*-3-(benzo[b]thiophen-6-yloxy)-9-azabicyclo[3.3.1]nonane and
*exo*-3-(benzo[b]thiophen-4-yloxy)-9-azabicyclo[3.3.1]nonane;
or a pharmaceutically acceptable salt or solvate thereof.

The 9-azabicyclo[3.3.1]nonane derivatives of Formula I are prepared by methods well known in the art of organic chemistry, see for example, J. March, 'Advanced Organic Chemistry' 4th Edition, John Wiley and Sons. During synthetic sequences it may be necessary and/or desirable to protect sensitive or reactive groups on any of the molecules concerned. This is achieved by means of conventional protecting groups, such as those described in T.W. Greene and P.G.M. Wuts 'Protective Groups in Organic Synthesis' 2nd Edition, John Wiley and Sons, 1991. The protective groups are optionally removed at a convenient subsequent stage using methods well known in the art.

Compounds of formula I, wherein X is O can be prepared by coupling of compounds of formula II, wherein R¹ has the meaning as previously defined and OY represents a hydroxy or activated derivative thereof such as mesylate or tosylate, with alcohols of formula ArOH wherin Ar has the meaning as previously defined. When -OY represents a suitable leaving group (*e.g*. mesylate or tosylate) nucleophilic displacement can be effected with the aid of a strong organic or inorganic base such as BEMP, potassium carbonate. Additionally nucleophilic displacement can be effected using a preformed metal salt, formed by reaction of alcohols of formula ArOH with a metal hydride, for example sodium hydride. When -OY represents a hydroxy (i.e. Y=H) the substitution reaction can also be effected using the Mitsunobu reaction with the aid of coupling reagents such as diethylazodicarboxylate(DEAD) and triphenylphosphine, 1,1'-azodicarbonyldipiperidine(ADDP) and tributylphosphine or (4,4-dimethyl-1,1-dioxido-1,2,5-thiadiazolidin-2-yl)triphenylphosphonium.

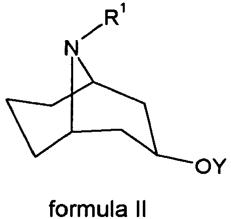

Compounds of the formula II can be prepared by literature procedures or modifications of literature procedures known to those persons skilled in the art. For example by reduction of a 9-azabicyclo[3.3.1]nona-3-one with a suitable reducing agent such as sodium borohydride in a suitable solvent such as ethanol.

Compounds of formula ArOH, wherin Ar has the meaning as previously defined, can be obtained from commercial sources, prepared by literature procedures or modifications of literature procedures known to those persons skilled in the art. For example, compounds of formula ArOH, wherein the aryl group is substituted with one or more halogens can be prepared by halogenation of the related methyl ethers (*i.e*. ArOMe) using procedures well known in the art followed by demethylation with, for example, pyridine hydrochloride. For example, chlorination can be accomplished using agents such as thionyl chloride, oxalyl chloride or N-chlorosuccinimide and bromination can be accomplished using phosphorous tribromide or a combination of carbon tetrabromide and triphenylphosphine.

Compounds of formula ArOH can also be prepared by standard functional group transformations well known in the art of organic chemistry, for example, from the corresponding nitroaryl (ArNO₂), aniline (ArNH₂), or methoxyaryl (ArOMe) precursors. For instance compounds of formula ArOH (IV) can be prepared via diazotisation of the corresponding aniline (III) using sodium nitrite and conc. sulphuric acid as shown in Scheme 1

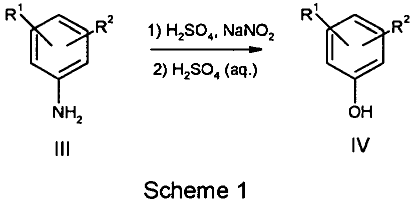

A further route to compounds of formula ArOH (IV) is via demethylation of the corresponding methoxyaryl derivative (V) with pyridine hydrochloride as shown in Scheme 2.

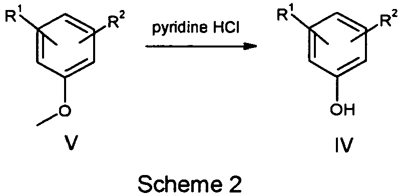

When Ar is benzo[b]thiophene, compounds of formula ArOH can be prepared by literature procedures or modifications of literature procedures known to persons skilled in the art, for example, the procedures disclosed in WO 04/043904. A preferred route to benzo[b]thiophenes is by the alkylation of a thiophenol derivative (VI) with bromoacetaldehyde diethyl acetal (VII) followed by acid catalysed deprotection and subsequent cyclisation with boron trifluoride etherate (Scheme 3). Demethylation of the methoxythiophene (X) provides the hydroxybenzo[b]thiophene (XI) needed for subsequent ether formation.

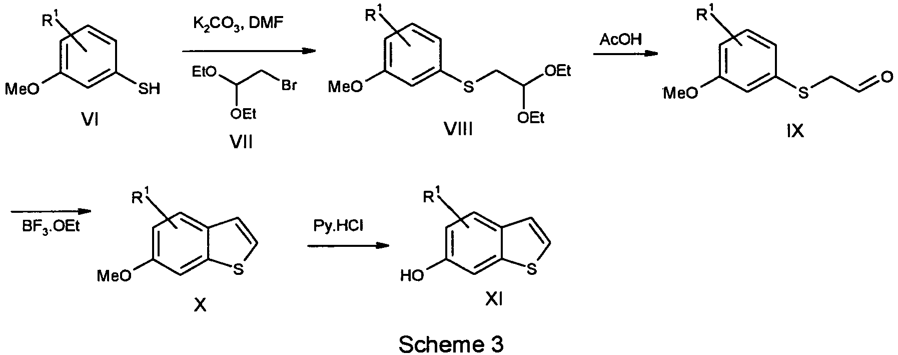

When Ar is benzo[d]isothiazole, compounds of formula ArOH can be prepared by literature procedures or modifications of literature procedures known to persons skilled in the art, for example, utilising procedures disclosed in WO 04/043904. A preferred route to 3-hydroxybenzo[d]isothiazoles is shown in Scheme 4. Treatment of the fluorobenzoic acid (XII) with, for example, oxalyl chloride or thionyl chloride, followed by treatment with ammonia provides the benzamide (XIII). Substitution with benzyl mercaptan then affords the adduct (XIV) which following subsequent cyclisation is converted to the 3-hydroxybenzo[d]isothiazole derivative (XV) needed for subsequent ether formation.

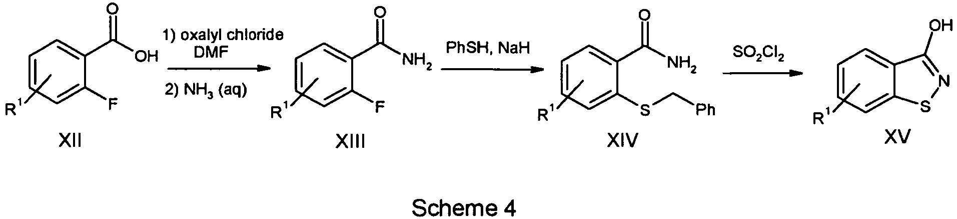

The present invention also includes within its scope all stereoisomeric forms of a 9-azabicyclo[3.3.1]nonane derivative as disclosed herein. In particular, the invention includes both *exo* and *endo* stereoisomers resulting when the 3-substituent is in the *exo* and *endo* configuration respectively. In the case of individual stereoisomers of compounds of formula I or salts or solvates thereof, the present invention includes the aforementioned stereoisomer substantially free, *i.e*., associated with less than 5%, preferably less than 2% and in particular less than 1 % of the other stereoisomer. Mixtures of stereoisomers in any proportions are also included within the scope of the present invention.

The present invention also includes within its scope all isotopically labelled forms of the compounds of the invention. For example, compounds isotopically labelled with ²H, ³H, ¹¹C, ¹³C, ¹⁴C, ¹³¹I, ¹²⁵I, ¹²³I and ¹⁸F. The labelled compounds are useful as diagnostic tools, radio tracers, or monitoring agents in various diagnostic methods and for *in vivo* receptor imaging.

The 9-azabicyclo[3.3.1]nonane derivatives of the present invention, in the form as a free base, are isolated from reaction mixtures as pharmaceuticaly acceptable salts. These salts are also obtained by treatment of said free base with an organic or inorganic acid such as hydrogen chloride, hydrogen bromide, hydrogen iodide, sulfuric acid, phosphoric acid, acetic acid, trifluoroacetic acid, propionic acid, glycolic acid, maleic acid, malonic acid, methanesulphonic acid, fumaric acid, succinic acid, tartaric acid, citric acid, benzoic acid and ascorbic acid. All salts, whether pharmaceutically acceptable or not are included within the scope of the present invention.

The 9-azabicyclo[3.3.1]nonane derivatives of the present invention exist in both solvated and unsolvated forms, including hydrated forms. Both these forms are encompassed within the scope of the present invention.

The 9-azabicyclo[3.3.1]nonane derivatives of the present invention also exist as amorphous forms. Multiple crystalline forms are also possible. All these physical forms are included within the scope of the present invention.

The 9-azabicyclo[3.3.1]nonane derivatives of the present invention are, *inter alia,* mixed neurotransmitter reuptake inhibitors as demonstrated *in vitro* by their ability to inhibit the reuptake of one or more of serotonin, noradrenaline and dopamine in cells stably transfected with, for example, the human serotonin, noradrenaline and dopamine transporters. Consequently, the 9-azabicyclo[3.3.1]nonane derivatives of the present invention are useful in therapy. As such, the 9-azabicyclo[3.3.1]nonane derivatives of the present invention are useful in the manufacture of a medicament for the treatment or prevention of diseases for which the reuptake inhibition of one or more monoamine neurotransmitters contributes to the therapeutic effect. In particular, the 8-azabicyclo[3.2.1]octane derivatives of the present invention are useful for the manufacture of a medicament for the treatment or prevention of diseases of the nervous system, both centrally and peripherally, for which the reuptake inhibition of one or more monoamine neurotransmitters contributes to the therapeutic effect.

In a further aspect the 9-azabicyclo[3.3.1]nonane derivatives of the present invention are useful for the treatment or prevention of depression, anxiety, pain, panic disorders and obsessive compulsive disorder. Depression states in the treatment of which the 8-azabicyclo[3.2.1]octane derivatives of the present invention and their pharmaceutically acceptable salts and solvates are particularly useful are those classified as mood disorders in the Diagnostic and Statistical Manual of Mental Disorders, Fourth Edition-Text Revised, American Psychiatric Association, Washington D.C. (2000), including mood episodes, depressive disorders, bipolar disorders and other mood disorders.

The present invention further includes a compound of formula (I) for use in a method for the treatment of a mammal, including a human, suffering from or liable to suffer from any of the aforementioned diseases or disorders, said method comprising administering an effective amount of a 9-azabicyclo[3.3.1]nonane derivative of the present invention or a pharmaceutically acceptable salt or solvate thereof.

The amount of a 9-azabicyclo[3.3.1]nonane derivative of the present invention or a pharmaceutically acceptable salt or solvate thereof, also referred to herein as the active ingredient, which is required to achieve a therapeutic effect will, of course, vary with the particular compound, the route of administration, the age and condition of the recipient, and the particular disorder or disease being treated.

A suitable daily dose for any of the above mentioned disorders will be in the range of 0.001 to 50 mg per kilogram body weight of the recipient (*e.g.* a human) per day, preferably in the range of 0.01 to 20 mg per kilogram body weight per day. The desired dose may be presented as multiple sub-doses administered at appropriate intervals throughout the day.

Whilst it is possible for the active ingredient to be administered alone, it is usual to present it as a pharmaceutical formulation. The present invention therefore also provides a pharmaceutical composition comprising a 9-azabicyclo[3.3.1]nonane derivative according to the present invention in admixture with one or more pharmaceutically acceptable excipients, such as the ones described in Gennaro et. al., Remmington: The Science and Practice of Pharmacy, 20th Edition, Lippincott, Williams and Wilkins, 2000; see especially part 5: pharmaceutical manufacturing. Suitable excipients are described *e.g*., in the Handbook of Pharmaceutical Excipients, 2nd Edition; Editors A. Wade and P.J.Weller, American Pharmaceutical Association, Washington, The Pharmaceutical Press, London, 1994. Compositions include those suitable for oral, nasal, topical (including buccal, sublingual and transdermal), parenteral (including subcutaneous, intravenous and intramuscular) or rectal administration.

The mixtures of a 9-azabicyclo[3.3.1]nonane derivative according to the present invention and one or more pharmaceutically acceptable excipient or excipients may be compressed into solid dosage units, such as tablets, or be processed into capsules or suppositories. By means of pharmaceutically suitable liquids the compounds can also be applied as an injection preparation in the form of a solution, suspension, emulsion, or as a spray, *e.g*., a nasal or buccal spray. For making dosage units *e.g*., tablets, the use of conventional additives such as fillers, colorants, polymeric binders and the like is contemplated. In general, any pharmaceutically acceptable additive can be used. The 9-azabicyclo[3.3.1]nonane derivatives of the present invention are also suitable for use in an implant, a patch, a gel or any other preparation for immediate and/or sustained release.

Suitable fillers with which the pharmaceutical compositions can be prepared and administered include lactose, starch, cellulose and derivatives thereof, and the like, or mixtures thereof used in suitable amounts.

The following Examples further illustrate the compounds of the present invention and methods for their synthesis.

In the following section, there is described the synthesis of precursors and common intermediates for compounds of the present invention.

### endo-3-Hydroxy-9-azabicyclo[3.3.1]nonane-9-carboxylic acid tert-butyl ester

### a) 9-Benzyl-9-azabicyclo[3.3.1]nonan-3-one

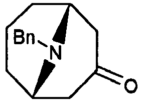

Glutaraldehyde (25% solution in water) (910 mL, 2.4 mol) and benzylamine hydrochloride (344.1 g, 2.4 mol) in water (1050 mL) was cooled to 0°C. 3-Oxopentanedioic acid (350.0g, 2.4 mol) was added followed by addition of sodium acetate (aq.) (79.7 g in 797 mL water) resulting in formation of a thick orange precipitate. The reaction mixture was heated to 50°C and stirred at this temperature for 4 h. It was then cooled to ambient temperature and allowed to stand for 24 h. The reaction mixture was acidified to pH2 with 5N aqueous hydrochloric acid (-150 mL) and the resulting aqueous mixture was washed with diethyl ether (2 x 500 mL). The aqueous extracts were basified to pH12 with 4N aqueous sodium hydroxide (-650 mL) and extracted with dichloromethane (6 x 500 mL). The organic phase was dried (MgSO₄) and concentrated under reduced pressure to afford the crude product as a red oil. Purification by chromatography on silica gel with dichloromethane:methanol (49:1, v/v) as eluent afforded *9-benzyl-9-azabicyclo[3.3.1]nonan-3-one* (300.0 g, 1.3 mol, 54%) as a pale orange solid.

### b) endo-9-Benzyl-9-azabicyclo[3.3.1]nonan-3-ol

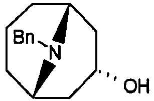

Sodium borohydride (6.22 g, 0.16 mol) was added portionwise (30 min) to a solution of 9-benzyl-9-azabicyclo[3.3.1]nonan-3-one (25.00 g, 0.11 mol) in methanol (130 mL) cooled to 0 °C under a nitrogen atmosphere. The reaction mixture was warmed to ambient temperature and stirring continued at this temperature for 12 h. The reaction mixture was quenched with acetone (10 mL) and volatiles evaporated *in vacuo.* The resultant yellow solid was dissolved in water (110 mL) and extracted with dichloromethane (3 x 40 mL). The organic phase was dried (MgSO₄) and evaporated under reduced pressure to afford crude *endo-9-benzyl-9-azabicyclo[3.3.1]nonan-3-ol* (25.25g, 0.11 mol, 100%) as a yellow solid.

### c) endo-9-Azabicyclo[3.3.1]nonan-3-ol

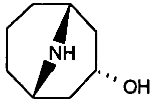

10% Palladium on carbon (10% Pd/C) (5.0 g) was added to a solution of *endo*-9-benzyl-9-azabicyclo[3.3.1]nonan-3-ol (27.0 g, 0.12 mol) in ethanol (500 mL) and 5N aqueous hydrochloric acid (25 mL). The mixture was stirred under a hydrogen atmosphere (3 bar) at 40°C for 48 h. The mixture was then filtered through a pad of dicalite and the filtrate was evaporated *in vacuo* and remaining aqueous mixture azeotroped with toluene (2 x 150 mL) to yield crude *endo-9-azabicyclo[3.3.1]nonan-3-ol* as a pale yellow solid which was used directly in the next stage.

### d) endo-3-Hydroxy-9-azabicyclo[3.3.1]nonane-9-carboxylic acid tert-butyl ester

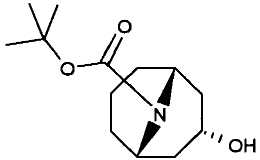

Crude *endo*-9-azabicyclo[3.3.1]nonan-3-ol (from Step c) was dissolved in a solution of dioxane (500 mL), water (187.5 mL) and 4N sodium hydroxide (aq.) (62.5 mL) and cooled to -15°C under a nitrogen atmosphere. Di-*tert*-butyl dicarbonate (39.0 g, 0.18 mol) was added portionwise over 10 min, the reaction mixture allowed to warm to ambient temperature and stirring continued for 4 h during which time a white precipitate formed. Dioxane was evaporated under reduced pressure. Water (400 mL) was added and the product extracted with dichloromethane (3 x 400 mL). The organic phase was dried (MgSO₄) and concentrated *in vacuo* to afford *endo-3-hydroxy-9-azabicyclo[3.3.1]nonane-9-carboxylic acid tert-butyl ester* (24.0 g, 0.10 mol, 83% from *endo*-9-benzyl-9-azabicyclo[3.3.1]nonan-3-ol) as a pale yellow solid.

### exo-3-Hydroxy-9-azabicyclo[3.3.1]nonane-9-carboxylic acid tert-butyl ester

### a) exo-9-Benzyl-9-azabicyclo[3.3.1]nonan-3-ol

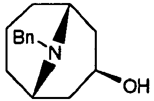

Sodium (1.6 g, 69.8 mmol) was added portionwise to a solution of 9-benzyl-9-azabicyclo[3.3.1]nonan-3-one (1.0 g, 4.36 mmol) in 1-pentanol (50 mL) heated at reflux temperature. The reaction mixture was heated at 136°C for 2 h followed by cooling to ambient temperature. 5N aqueous HCl (50 mL) was added, the aqueous layer separated and the organic phase extracted with further 5N aqueous HCl (5 x 50 mL). The aqueous extracts were washed with diethyl ether (3 x 70 mL) and basified by addition of 10N aqueous KOH (taken to pH8) followed by saturated aqueous K₂CO₃. The aqueous mixture was extracted with dichloromethane (5 x 40 mL) and the organic phase washed with brine (60 mL), dried (Na₂SO₄) and concentrated *in vacuo* to afford *exo-9-benzyl-9-azabicyclo[3.3.1]nonan-3-ol* (1.93 g, 96%) as a brown oil.
Data for *exo-9-benzyl-9-azabicyclo[3.3.1]nonan-3-ol: :* MS (ESI) *m*/*z*: 232 ([M+H]+).

### b) exo-9-Azabicyclo[3.3.1]nonan-3-ol

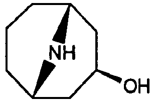

The procedure for the preparation of *endo*-9-azabicyclo[3.3.1]nonan-3-ol was followed to afford *exo-9-azabicyclo[3.3.1]nonan-3-ol* (5.0 g, 100%) as a pale yellow oil. Data for *exo-9-azabicyclo[3.3.1]nonan-3-ol::* MS (ESI) *mlz:* 142 ([M+H]+).

### c) exo-3-Hydroxy-9-azabicyclo[3.3.1]nonane-9-carboxylic acid tert-butyl ester

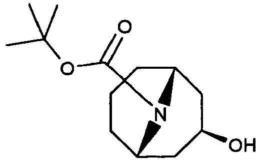

The procedure for the preparation of *endo*-3-hydroxy-9-azabicyclo[3.3.1]nonane-9-carboxylic acid *tert*-butyl ester was followed to afford *exo-3-hydroxy-9-azabicydo[3.3.1]nonane-9-carboxylic acid tert-butyl ester* (663 mg, 71 %) as a pale yellow oil.

### Benzo[b]thiophene-6-ol

### a) 1-(2,2-Diethoxyethylsulphanyl)-3-methoxybenzene

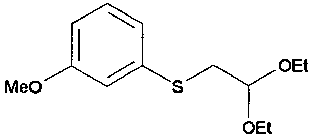

3-Methoxythiophenol (10.00 g, 71.33 mmol), bromoacetaldehyde diethyl acetal (11.47 mL, 76.24 mmol), potassium carbonate (10.35 g, 74.89 mmol) and acetone (100 mL) were stirred at ambient temperature under nitrogen for 3 h. The white suspension was filtered, washed with acetone (2 x 25 mL) and the combined filtrate and washings concentrated to dryness *in vacuo.* The residue was dissolved in ethyl acetate (50 mL) and washed with 0.5N NaOH (aq.) (2 x 1 5 mL), water (15 mL) and brine (15 mL). The organic layer was dried (MgSO₄) and concentrated *in vacuo* and then further dried at 130°C under high vacuum to give the title compound (18.74 g, quantitative).

### b) (3-Methoxyphenylsulphanyl)acetaldehyde

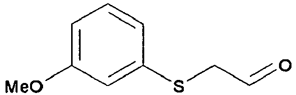

1-(2,2-Diethoxyethylsulphanyl)-3-methoxybenzene (18 g, 70.21 mmol), glacial acetic acid (26 mL) and water (18 mL) were stirred at reflux temperature under nitrogen for 45 min. The reaction mixture was cooled to ambient temperature, diluted with water (180 mL) and extracted with *tert*-butyl methyl ether (3 x 40 mL). The combined extracts were cooled to <5°C and washed with 5% aqueous sodium carbonate solution, water, and brine. The organic phase was dried (MgSO₄) and concentrated *in vacuo* to yield the title compound (11.8 g, 92 %).

### c) 6-Methoxybenzo[b]thiophene

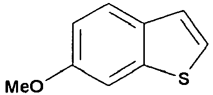

Boron trifluoride etherate (8.15 mL) dissolved in dry dichloromethane (407 mL) was stirred rapidly at 0°C under nitrogen and a solution of (3-methoxyphenylsulphanyl)acetaldehyde (11.5 g, 63.10 mmol) in dry dichloromethane (29 mL) was added dropwise over 25 min.

The resultant green solution was stirred for 2 min and then saturated aqueous sodium bicarbonate solution (150 mL) was added at a rate so as to maintain the temperature <8°C. The reaction mixture was stirred for 5 min and then the layers were separated and the organic layer was washed with saturated aqueous sodium bicarbonate solution (100 mL) and water (100 mL). The organic phase was dried (MgSO₄), and concentrated *in vacuo.* The product was purified by distillation to afford the title compound as a colourless oil (6.29 g, 61 %, b.p. 83-88 °C at 16 mBar).

### d) Benzo[b]thiophen-6-ol

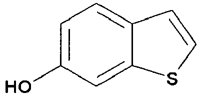

6-Methoxybenzo[b]thiophene (6.15 g, 37.45 mmol) and pyridine hydrochloride (18.5 g) were heated together at 210°C for 1 h. The mixture was cooled to 100°C, diluted with water (125 mL) and extracted with dichloromethane (3 x 70 mL). The combined extracts were washed with water (125 mL), dried (MgSO₄) and evaporated *in vacuo* to give the title compound (5.54 g, 98 %)

### Procedure I

### EXAMPLE I.1: exo-3-(Benzo[d]isothiazol-7-yloxy)-9-azabicyclo[3.3.1]nonane

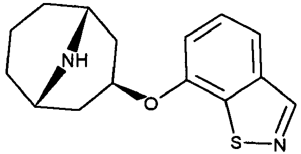

*Endo*-3-hydroxy-9-azabicyclo[3.3.1]nonane-9-carboxylic acid *tert*-butyl ester (241 mg, 1.00 mmol), (4,4-dimethyl-1,1-dioxido-1,2,5-thiadiazolidin-2-yl)triphenyl phosphonium (prepared as described in J. Org. Chem., 1994, 59, 2289) (616 mg, 1.50 mmol) and benzo[d]isothiazol-7-ol (prepared as described in WO 04/043904) (151 mg, 1.00 mmol) were dissolved in dry THF (5 mL) in a microwave vial. The reaction was heated at 140 °C for 10 min in the microwave oven. On cooling the solvent was evaporated under reduced pressure. The product was purified by chromatography on silica gel with a gradient of dichloromethane to dichloromethane:methanol (49:1, v/v) to dichloromethane:methanol (19:1, v/v) as eluent to afford crude product (230 mg, 84%). The crude product was dissolved in a solution of trifluroacetic acid (TFA) (1 mL) and dichloromethane (5 mL) and stirred at ambient temperature for 1 h. Volatiles were evaporated under reduced pressure, the residue dissolved in methanol (1 mL) and passed through an SCX cartridge (primed with dilute TFA/methanol), washed with methanol, then product eluted with a solution of 2M ammonia in methanol. The product was further purified by chromatography on slica gel with dichloromethane:methanol (49:1, v/v) to dichloromethane:methanol (19:1, v/v) as eluent to afford *exo-3-(benzo[d]isothiazol-7-yloxy)-9-azabicyclo[3.3.1]nonane* (90 mg, 53 %).

Data for *exo-3-(benzo[d]isothiazol-7-yloxy)-9-azabicyclo[3.3.1]nonane:* MS (ESI) *m*/*z*: 275 ([M+H]⁺).

Similarly prepared were:

### EXAMPLE I.2: exo-3-(Benzo[d]isothiazol-4-yloxy)-9-azabicyclo[3.3.1]nonane

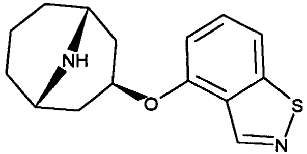

Prepared from benzo[d]isothiazol-4-ol (prepared as described in WO 04/043904). MS (ESI) (*m*/*z*): 275 ([M+H]⁺).

### Procedure II

### EXAMPLE II.1: exo-3-(3-Chloro-2-fluorophenoxy)-9-azabicyclo[3.3.1]nonane

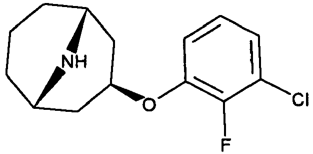

*Endo*-3-hydroxy-9-azabicyclo[3.3.1]nonane-9-carboxylic acid *tert*-butyl ester (150 mg, 0.62 mmol), (4,4-dimethyl-1,1-dioxido-1,2,5-thiadiazolidin-2-yl)triphenyl phosphonium (prepared as described in J. Org. Chem., 1994, 59, 2289) (383 mg, 0.93 mmol) and 3-chloro-2-fluorophenol (137 mg, 0.93 mmol) were dissolved in dry THF (4 mL). The reaction mixture was stirred at ambient temperature for 4 h. The solvent was evaporated under reduced pressure, the residue dissolved in dichloromethane (5 mL) and trifluoroacetic acid (2 mL). Stirring was continued for 2 h. Volatiles were evaporated under reduced pressure, the residue dissolved in methanol (1 mL) and passed through an SCX cartridge (primed with dilute TFA/methanol), washed with methanol, then the product was eluted with a solution of 2M ammonia in methanol. The product was further purified by chromatography on silica gel with dichloromethane:methanol:ammonia (aq.) (89.9:10:0.1, v/v) as eluent to afford *exo-3-(3-chloro-2-fluorophenoxy)-9-azabicyclo[3.3.1]nonane (45* mg, 27 %).

Data for *exo-3-(3-chloro-2-fluorophenoxy)-9-azabicyclo[3.3.1]nonane:* MS (ESI) *m*/*z*: 270/272 ([M+H]⁺).

Similarly prepared were:

### EXAMPLE II.33: exo-3-(Benz[b]thiophen-7-yloxy)-9-azabicyclo[3.3.1]nonane

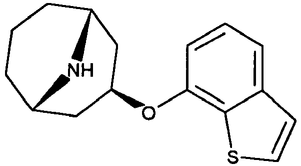

Prepared from benz[b]thiophen-7-ol (prepared as described in J. Chem. Soc. (Perkin Trans. 1), 1993, 2973). MS (ESI) *m*/*z*: 274 ([M+H]⁺).

### EXAMPLE II.35: exo-3-(Benz[b]thiophen-6-yloxy)-9-azabicyclo[3.3.1]nonane

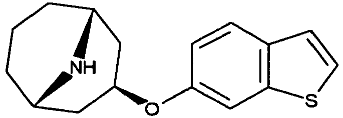

Prepared from benz[b]thiophen-6-ol. MS (ESI) *m*/*z*: 274 ([M+H]⁺).

### EXAMPLE II.36: exo-3-(Benzo[b]thiophen-4-yloxy)-9-azabicyclo[3.3.1]nonane

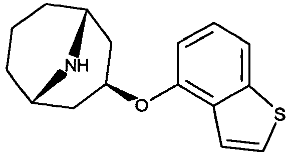

Prepared from benzo[b]thiophen-4-ol (prepared as described in J. Chem. Res., 1993, 192). MS (ESI) *m*/*z*: 274 ([M+H]⁺).

### Method XI: Assay of monoamine uptake

The *in vitro* test for the inhibition of dopamine and serotonin uptake was performed in Chinese Hamster Ovary cells expressing the human dopamine transporter (hDAT) or the human serotonin transporter (hSERT). The *in vitro* test for the inhibition of noradrenaline uptake was performed in Madin Darby Canine Kidney Cells (MDCK) expressing the human noradrenaline transporter (hNET).

Briefly, cell lines stably overexpressing the appropriate human transporter were propagated and plated according to standard cell culture techniques. Following plating, cells were left to adhere for either one or two days. A 6-point serial dilution (normally 1 E-5M to 1E-10M) of test and reference compounds was prepared, added to the washed cells and incubated for 5 minutes at room temperature for dopamine or serotonin transporter overexpressing and 37°C for noradrenaline overexpressing cells. Next, a final concentration of 20 nM of appropriate neurotransmitter (mixture of [³H]-neurotransmitter and non-labelled neurtoransmitter) was added and the cells were incubated for three or five minutes at room temperature for dopamine or serotonin transporter overexpressing cells or ten minutes at 37°C for noradrenaline overexpressing cells. Following termination of the assay, Microscint-20 was added directly to the cells and the amount of radioactivity taken up by the cells was estimated by scintillation counting.

EC₅₀ values indicating inhibition of monoamine uptake were calculated using standard curve fitting techniques.

## Claims

1. A 9-azabicyclo[3.3.1]nonane derivative of formula I, 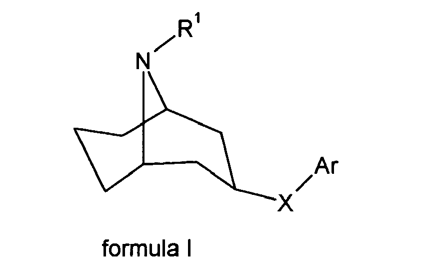 wherein
R¹ is H;
X is O and
Ar is a heteroaryl ring selected from benzothienyl and benzoisothiazolyl,
said 9-azabicyclo[3.3.1]nonane derivative being selected from:
*exo*-3-(benzo[d]isothiazol-7-yloxy)-9-azabicyclo[3.3.1]nonane;
*exo*-3-(benzo[d]isothiazol-4-yloxy)-9-azabicyclo[3.3.1]nonane;
*exo*-3-(benzo[b]thiophen-7-yloxy)-9-azabicyclo[3.3.1]nonane;
*exo*-3-(benzo[b]thiophen-6-yloxy)-9-azabicyclo[3.3.1]nonane and
*exo*-3-(benzo[b]thiophen-4-yloxy)-9-azabicyclo[3.3.1]nonane;
or a pharmaceutically acceptable salt or solvate thereof.

2. A 9-azabicyclo[3.3.1]nonane derivative according to claim 1 for use in therapy.

3. A pharmaceutical composition comprising a 9-azabicyclo[3.3.1]nonane derivative according to claim 1 in admixture with one or more pharmaceutically acceptable excipient.

4. Use of a 9-azabicyclo[3.3.1]nonane derivative according to claim 1 for the manufacture of a medicament for the treatment or prevention of a disease or disorder for which the reuptake inhibition of one or more monoamine neurotransmitter contributes to the therapeutic effect.

5. Use according to claim 4, wherein the medicament is for the treatment or prevention of depression or pain.

## Patentansprüche

1. Ein 9-Azabicyclo[3.3.1]nonan-Derivat der Formel I, 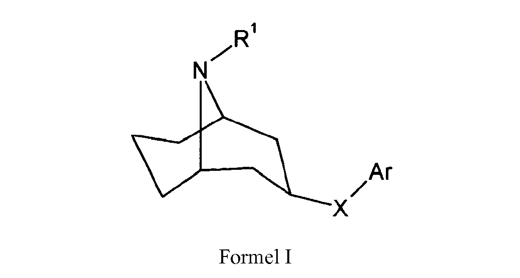 wobei
R¹ H ist,
X O ist und
Ar ein Heteroarylring ist, ausgewählt aus Benzothienyl und Benzoisothiazolyl,
wobei das 9-Azabicyclo[3.3.1]nonan-Derivat ausgewählt ist aus:
*exo*-3-(Benzo[d]isothiazol-7-yloxy)-9-azabicyclo[3.3.1]nonan,
*exo*-3-(Benzo[d]isothiazol-4-yloxy)-9-azabicyclo[3.3.1]nonan,
*exo*-3-(Benzo[b]thiophen-7-yloxy)-9-azabicyclo[3.3.1]nonan,
*exo*-3-(Benzo[b]thiophen-6-yloxy)-9-azabicyclo[3.3.1]nonan und
*exo*-3-(Benzo[b]thiophen-4-yloxy)-9-azabicyclo[3.3.1]nonan,
oder ein pharmazeutisch annehmbares Salz oder Solvat davon.

2. Ein 9-Azabicyclo[3.3.1]nonan-Derivat gemäß Anspruch 1 zur Verwendung in der Therapie.

3. Eine pharmazeutische Zusammensetzung, die ein 9-Azabicyclo[3.3.1]nonan-Derivat gemäß Anspruch 1 im Gemisch mit einem oder mehreren pharmazeutisch annehmbaren Hilfsstoffen umfasst.

4. Verwendung eines 9-Azabicyclo[3.3.1]nonan-Derivats gemäß Anspruch 1 zur Herstellung eines Medikaments zur Behandlung oder Prävention einer Erkrankung oder eines Zustandes, bei dem die Wiederaufnahmeinhibierung von einem oder mehreren Monoamin-Neurotransmittern zum therapeutischen Effekt beiträgt.

5. Verwendung gemäß Anspruch 4, wobei das Medikament zur Behandlung oder Prävention von Depression oder Schmerzen dient.

## Revendications

1. Dérivé de 9-azabicyclo [3.3.1]nonane de la formule I, 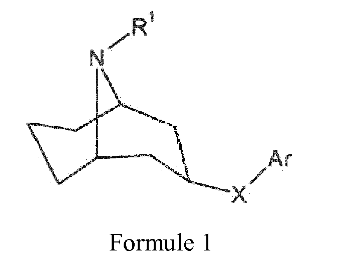 où:
R¹ est H;
X est O et
Ar est un cycle hétéroaryle sélectionné parmi le benzothiényle et le benzoisothiazolyle,
ledit dérivé de 9-azabicyclo[3.3.1]nonane étant sélectionné parmi:
l'*exo*-3-(benzo[d]isothiazol-7-yloxy)-9-azabicyclo[3.3.1]nonane;
l'*exo*-3-(benzo[d]isothiazol-4-yloxy)-9-azabicyclo[3.3.1]nonane;
l'*exo*-3-(benzo[b]thiophén-7-yloxy)-9-azabicyclo[3.3.1]nonane;
l'*exo*-3-(benzo[b]thiophén-6-yloxy)-9-azabicyclo[3.3.]nonane et
l'*exo*-3-(benzo[b]thiophén-4-yloxy)-9-azabicyclo[3.3.1]nonane;
ou un sel pharmaceutiquement acceptable de celui-ci.

2. Dérivé de 9-azabicyclo[3.3.1]nonane selon la revendication 1, à utiliser en thérapie.

3. Composition pharmaceutique comprenant un dérivé de 9-azabicyclo[3.3.1]nonane selon la revendication 1, en addition/mélange avec un excipient pharmaceutiquement acceptable ou plus.

4. Utilisation d'un dérivé de 9-azabicyclo[3.3.1]nonane selon la revendication 1, dans la fabrication d'un médicament pour le traitement ou la prévention d'une maladie ou d'un trouble pour lequel inhibition du recaptage d'un ou de plusieurs neurotransmetteurs monoamines contribue à l'effet thérapeutique.

5. Utilisation selon la revendication 4, où le médicament est destiné au traitement ou à la prévention de la dépression ou de la douleur.
